(19) Europäisches Patentamt
European Patent Office
Office européen des brevets

(11) Veröffentlichungsnummer: **0 207 463**
**A2**

(12) # EUROPÄISCHE PATENTANMELDUNG

(21) Anmeldenummer: **86108781.5**

(51) Int. Cl.⁴: **C07H 15/252**, A61K 31/70

(22) Anmeldetag: **04.07.86**

(30) Priorität: **05.07.85 DE 3524117**

(43) Veröffentlichungstag der Anmeldung:
**07.01.87 Patentblatt 87/02**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Berscheid, Hans-Gerd, Dr.**
**Rheinlandstrasse 21**
**D-6231 Schwalbach am Taunus(DE)**
Erfinder: **Böttger, Dirk, Dr.**
**Bahnstrasse 4**
**D-6237 Liederbach(DE)**
Erfinder: **Kraemer, Hans Peter, Dr.**
**Birkenweg 16**
**D-3550 Marburg(DE)**

(54) **Neue Anthracyclin-Tetrasaccharide, ein Verfahren zu ihrer Herstellung und ihre Verwendung als Cytostatika.**

(57) Die vorliegende Erfindung betrifft Anthracyclin-Derivate und ein Verfahren zu ihrer Herstellung. Die Verbindungen zeichnen sich durch eine hohe cytostatische Wirksamkeit aus und können daher als Arzneimittel mit Anti-Tumor-Wirkung verwendet werden.

EP 0 207 463 A2

# Neue Anthracyclin-Tetrasaccharide, ein Verfahren zu ihrer Herstellung und ihre Verwendung als Cytostatika

Die vorliegende Erfindung betrifft Anthracyclin-Derivate der Formel I

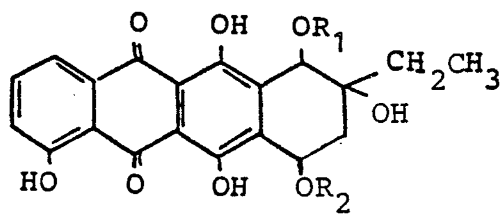

(I),

worin $R_1$ und $R_2$ verschieden sind und einer der beiden Reste den Zuckerbaustein L-Rhodosamin - (Roa) der Formel

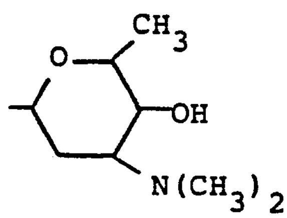

darstellt, während der andere die Zuckerkombination L-Rhodosamin-L-Rhodinose-L-Rhodinose (Roa-Rod-Rod) der Formel

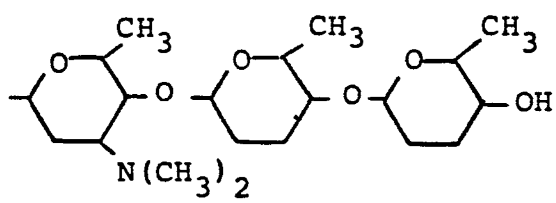

oder L-Rhodosamin-L-Rhodinose-L-Aculose (Roa-Rod-Acu) der Formel

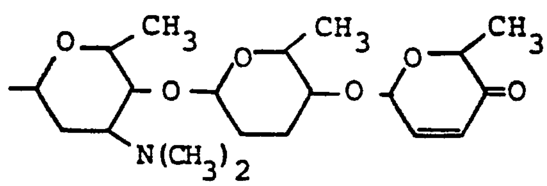

oder L-Rhodosamin-2-desoxy-L-Fucose-L-Cinerulose A (Roa-dF-Cin-A) der Formel

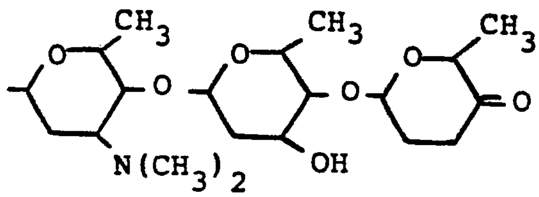

darstellt.

Die vorliegende Erfindung betrifft ferner ein Verfahren zur Herstellung der Verbindungen der Formel I, das dadurch gekennzeichnet ist, daß man in einer der in EP-A-0 131 181 (HOE 83/F 103) beschriebenen Verbindungen der Formel II

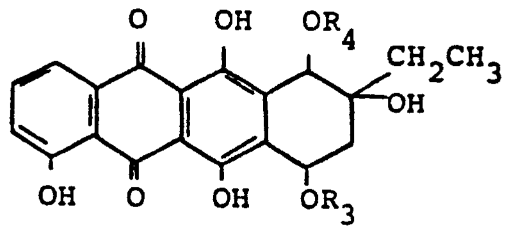

(II),

worin einer der beiden Substituenten $R_3$ und $R_4$ eine der Zuckerkombinationen Roa-Rod-Rod, Roa-Rod-Acu, oder Roa-df-CinA und der andere Substituent eine der Zuckerkombinationen Roa-Rod-Rod, Roa-Rod-Acu, Roa-dF-CinA, Roa-dF-Rod oder Roa-dF=CinB bedeutet, wobei CinB Cinerulose B darstellt und dF=CinB bedeutet, daß die beiden Zuckerbausteine durch eine zusätzliche Ätherbrücke neben der üblichen glykosidischen Bindung verknüpft sind, durch Behandlung mit einer Säure das endständige Disaccharid in einer der beiden Trisaccharidketten abspaltet.

Gemäß der EP-A-0 131 181 wird der Stamm Streptomyces purpurascens DSM 2658 in einem Nährmedium auf übliche Weise fermentiert, wobei Temperaturen von ca. 24-40°C, ein pH-Wert von 6,5 -8,5 und aerobe Bedingungen eingehalten werden. Aus dem Mycel werden die Anthracyclin-Verbindungen durch Extraktion z.B. mit wäßrigem Aceton bei pH 3,5 extrahiert, das Aceton entfernt und die wäßrige Phase bei einem pH-Wert von 7,5 mit Ethylacetat extrahiert. Die Kulturflüssigkeit extrahiert man bei einem pH-Wert von 7,5, vorzugsweise mit Ethylacetat. Die Ethylacetatextrakte aus dem Mycel und dem Kulturfiltrat werden vereinigt und ergeben, zur Trockne eingedampft, einen rohen Rückstand. Mann kann nun den so erhaltenen Rückstand, wie in der Europäischen Patentanmeldung EP-A-0 131 942 (HOE 83/F 139) beschrieben, einer Säurebehandlung unterziehen. Vorzugsweise arbeitet man jedoch den Rückstand weiter auf, wie in der EP-A-0 131 181 beschrieben.

Demzufolge wird der rohe Rückstand in Toluol gelöst und mit einem Acetatpuffer (pH 3,5) extrahiert, wobei man eine Toluolphase und eine wäßrige Phase erhält. Die wäßrige Phase wird weiter aufgearbeitet in der folgenden Weise: Nach Einstellen des pH-Wertes auf 7,5 wird wiederum mit Ethylacetat extrahiert und die Ethylacetatphase konzentriert, wobei man ein sogenanntes Cytorhodin-Roh-gemisch erhält. Gemäß der genannten Europäischen Patentammeldung können aus diesem Rohgemisch auf chromatographischem Wege Anthracyclin-Hexasaccharide der Formel II gewonnen werden.

In der EP-A-0 131 142 (HOE 83/F 139) wird beschrieben, daß man zu neuen, ebenso wirksamen Anthracyclin-Tetrasacchariden gelangt, wenn man den oben beschriebenen rohen Rückstand von Anthracyclin-Hexasacchariden, das sogenannte Cytorhodin-Rohgemisch, mit Säure behandelt. Überraschenderweise gelingt es nun, bei der Säurebehandlung von isolierten reinen Komponenten, die in der EP-A 0 131 181 beschrieben wurden, weitere neue, bisher nicht bekannte Hydrolyseprodukte mit Anthracyclin-Tetrasaccharid-Struktur zu gewinnen.

Als Säuren eignen sich z. B. verdünnte Salzsäure, Schwefelsäure, Phosphorsäure, Ameisensäure, Essigsäure und andere Säuren, gegebenenfalls auch nach Zusatz eines mit Wasser mischbaren organischen Lösungsmittels, z. B. Methanol oder Aceton. Man kann die Säurebehandlung auch mit stark sauren Ionenaustauschern in der H-

Form wie z.B. Dowex 50x8 ($H^+$) durchführen. In diesem Fall muß das gewünschte Produkt durch Elution vom Ionenaustauscher, z.B. mit Salzlösungen, gewonnen werden.

Die Säurebehandlung wird bei Raumtemperatur oder leicht erhöhter Temperatur, vorzugsweise 37°C, durchgeführt. Die Reaktionszeit kann 0,5 bis mehrere Stunden betragen, ist jedoch für jede Verbindungen unterschiedlich, wie durch Versuche zur Reaktionskinetik festgestellt wurde. Nach erfolgter Reaktion werden die gewünschten Verbindungen durch Extraktion aus dem Reaktionsmedium isoliert und durch Säulenchromatographie oder präparative Schichtchromatographie, vorzugsweise an Kieselgel, getrennt.

Man erhält durch das erfindungsgemäße Verfahren z.B. die folgenden Verbindungen, bei denen es sich um rote, amorphe Substanzen handelt, die leicht löslich in Methanol, Ethylacetat, Chloroform und Toluol, unlöslich hingegen in Wasser und Hexan sind und die dünnschichtchromatographisch an Kieselgel 60 $F_{254}$ (Merck) vorzugsweise mit dem Laufmittelsystem Chloroform/Methanol = 8/2 nachgewiesen werden können.

Cytorhodin SE

Rf-Wert im genannten System: 0,69

UV-Maxima: 234, 252(Sch), 296 und 496 nm in Methanol/10% 1 N-HCl

NMR-Spektrum: Abb. 1

$C_{48}H_{64}N_2O_{16}$ M ber. 924 (FAB-MS bestätigt)

Cytorhodin SE besitzt die Formel I, worin $R_1$ = Roa und $R_2$ = Roa-Rod-Acu bedeutet.

Cytorhodin TF

Rf-Wert im genannten System: 0,49

UV-Maxima: 235, 252(Sch), 296 und 496 nm in Methanol/10% 1 N-HCl

NMR-Spektrum: Abb. 2

$C_{48}H_{64}N_2O_{16}$ M ber. 924 (FAB-MS bestätigt)

Cytorhodin TF besitzt die Formel I, worin $R_1$ = Roa-Rod-Acu und $R_2$ = Roa bedeutet.

Cytorhodin SD

Rf-Wert im genannten System: 0,61

UV-Maxima: 234, 235(Sch), 292 und 495 nm in Methanol/10% 1 N-HCl

NMR-Spektrum: Abb. 3

$C_{48}H_{66}N_2O_{17}$, M ber. 942 (FAB-MS bestätigt)

Cytorhodin SD besitzt die Formel I, worin $R_1$ = Roa und $R_2$ = Roa-dF-CinA bedeutet.

Die erfindungsgemäßen Cytorhodine zeichnen sich durch eine hohe cytostatische Wirksamkeit aus und zeigen im Zellproliferationstest an L1210-Leukämiezellen folgende Wirkung:

| | $IC_{50}$ (um/ml) |
|---|---|
| Cytorhodin SE-Gluconat | $6 \times 10^{-3}$ |
| Cytorhodin TF-Gluconat | $2 \times 10^{-2}$ |
| Cytorhodin SD-Gluconat | $2 \times 10^{-1}$ |

Die Bestimmung der Hemmwirkung - (Zellproliferationstest) erfolgte wie nachstehend beschrieben:

L 1210 Zellen in exponentieller Wachstumsphase ($5x10^3$/ml in RPMI 1640) werden in einer Mikrotiterplatte 72 Stunden mit unterschiedlichen Konzentrationen der Testsubstanz inkubiert (37°C, 5 % $CO_2$, 95 % relative Luftfeuchte). Kontrolle bestehen aus Zellen, die lediglich mit frischem Medium inkubiert werden. Alle Bestimmungen werden als 4-fache Bestimmungen durchgeführt. Nach 65 Stunden werden 50 $\mu$l C14-Thymidin (1,5 $\mu$C/ml) zugegeben, um die DNA der Zelle radioaktiv zu markieren. Nach 7 Stunden Inkubation werden die Zellen abgesaugt, die DNA mit 5 %iger Trichloressigsäure gefällt und nacheinander mit Wasser bzw. Methanol gewaschen. Nach Trocknung bei 50°C wird die in die DNA eingebaute Radioaktivität nach Zugabe von 5 ml Szintillationsflüssigkeit ermittelt. Die Ergebnisse werden

angegeben als Verhältnisse des Szintillationsindex nach Inkubation mit mit Testsubstanz in Prozent der unbehandelten Kontrolle. Aus den so erhaltenen Meßwerten wird die Dosiswirkungskurve ermittelt und grafisch die $IC_{50}$. d.h. die Konzentration, die unter Testbedingungen den Einbau von radioaktivem Thymidin um 50 % gegenüber der Kontrolle erniedrigt, ermittelt.

Wie vorstehend ausgeführt, besitzen die erfindungsgemäßen Verbindungen zytostatische Wirksamkeit, d.h. therapeutische Wirkung gegen Tumoren, insbesondere maligne Tumoren bei Tieren und Menschen.

Die Verbindungen und die Säureadditionssalze können daher als Medikamente zur Behandlung von Tumoren verwendet werden. Die Verbindungen können auf verschiedene Weise in Abhängigkeit von der Dosierungsform verabreicht werden. Normalerweise werden die Verbindungen mit pharmazeutisch üblichen Trägerstoffen oder Verdünnungsmitteln vermischt verabreicht. So können sie z.B. einzeln oder im Mischung zusammen mit Trägerstoffen wie Maltose oder Lactose oder als nicht-toxische Komplexe, z.B. als Desoxyribobukleinsäure-Komplex verabreicht werden.

Eine typische Verabreichungsart ist die Injektion einer Lösung der erfindungsgemäßen Verbindung in destilliertem Wasser oder in physiologischer Kochsalzlösung. Die Lösungen können intraperitoneal, intravenös oder intraarteriell injiziert werden.

Tagesdosis und Einheitsdosis können aus Tierversuchen und auch aus in vitro-Tests in der Weise festgesetzt werden, daß die Gesamtdosis, die kontinuierlich oder in Abständen verabreicht wird, einen vorher festgelegten Bereich nicht überschreitet. So beträgt die Gesamtdosis für einen Behandlungszyklus etwa, 0,5-5 mg/kg Körpergewicht. Diese Dosis kann in entsprechenden Bruchteilen über einen Zeitraum von 7 Tagen verabreicht werden. Es ist jedoch klar, daß konkrete Dosen für die Behandlung von Mensch oder Tier individuell festgelegt werden können in Abhängigkeit von der jeweiligen Situation, z.B. Alter, Körpergewicht, Geschlecht, Empfindlichkeit, Nahrung, Zeitpunkt der Verabreichung, weiteren verabreichten Medikamenten, körperlichem Zustand der Patienten und Schwere der Erkrankung.

Die Herstellung der erfindungsgemäßen Verbindungen wird in nachfolgenden Beispielen beschrieben:

Beispiel 1

1,1 g Cytorhodin F wurden in 250 ml 0,1 N-Salzsäure gelöst und 115 Minuten bei Raumtemperatur gerührt. Mit 1 N-Natronlauge wurde ein pH-Wert von 3 eingestellt und dreimal mit je 250 ml Chloroform extrahiert. Bei pH 5,5 und pH 7, die ebenfalls durch Zusatz von 1 N-Natronlauge eingestellt wurden, erfolgte analog die Extraktion mit dreimal je 250 ml Chloroform. Die Extrakte bei pH 3 und pH 7 enthielten unumgesetztes Edukt sowie β-Rhodomycin II, welches als Folgeprodukt der Hydrolyse gebildet wird und von Brockmann et al. bereits beschrieben wurde (Naturwissenschaften 42, 492 (1950); Chem Ber. 86 , 261 (1953)). Die bei pH 5,5 extrahierte Lösung wurde über Natriumsulfat getrocknet und zu einem Rückstand von 380 mg eingeengt.

Der Rückstand wurde an 70 g Kieselgel (60, 15-40 µm, Merck) über eine Mitteldrucksäule 102 ml (Labomatic) chromatographiert. Hierzu wurde das Lösungsmittelgemisch Chloroform/Methanol/Eisessig/Wasser = 70/20/10/2 verwendet. Bei einer Fließgeschwindigkeit von 2 ml/min wurden 190 Fraktionen à 5 ml aufgefangen, die nach Prüfung über analytische HPLC - (LiChrosorb Si60, 5µm, Merck, mit dem System Chloroform/Methanol/ Eisessig/Wasser/Triethylamin = 750/100/100/20/0,5) folgendermaßen vereinigt wurden:

| | | | |
|---|---|---|---|
| Fr. 58 - 88 | 45 mg | Cytorhodin SE |
| Fr. 89 - 119 | 40 mg | Cytorhodine SE und TF |
| Fr. 120 - 190 | 81 mg | Cytorhodin TF |

Die vereinigten Fraktionen wurden zur Gewinnung der Trockensubstanzen bis zur Abscheidung der Chloroformphase mit einer 5%igen wäßrigen Lösung von $Na_2HPO_4$ versetzt, die Chloroformphase wurde mit einem Volumen $Na_2HPO_4$-Lösung und mit Wasser gewaschen, über Natriumsulfat getrocknet, im Vakuum eingeengt und mit Hexan gefällt.

Für die Testung der cytostatischen Aktivität wurden die Reinsubstanzen mit Gluconsäurelacton in die entsprechenden gut wasserlöslichen D-Glugonate überführt.

Beispiel 2

100 mg Cytorhodin P wurden in 10 ml, 0,1 N-HCl gelöst und bei Raumtemperatur 3,5 Stunden gerührt, die Reaktionslösung anschließend mit 0,1 N-NaOH auf pH 7,5 eingestellt und dreimal mit je 10 ml $CHCl_3$ geschüttelt. Die vereinigten organischen Phasen wurden mit wenig Wasser neutral gewaschen, über Natriumsulfat getrocknet und im Vakuum eingedampft.

Der trockene Rückstand (68 mg) enthielt nach Dünnschichtchromatogramm neben dem polaren β-Rhodomycin II zwei weniger polare Hauptprodukte und wurde durch präparative Schichtchromatographie aufgetrennt. Dazu wurden je 10 mg des Rückstandes in $CHCl_3$ gelöst auf eine DC-Alufolie Kieselgel 60, 0,2 mm, 20 cm x 20 cm, Merck, aufgetragen und mit der Unterphase des Lösungsmittelgemisches $CHCl_3$/Methanol/Wasser 130/40/70 chromatographiert.

Die herausgeschabten Banden wurden mit $CHCl_3$/Methanol 1:1 eluiert, das Eluat i.V. eingedampft. Nach Aufnehmen in wenig $CHCl_3$ wurde über eine Glasfritte filtriert und i.V. zur Trockne eingedampft. Es wurden 5 mg Cytorhodin SD und 2,5 mg Cytorhodin TF erhalten.

Cytorhodin SD lieferte mit D-Gluconsäurelacton das gut wasserlösliche D-Gluconat.

## Ansprüche

1. Verbindungen der allgemeinen Formel I

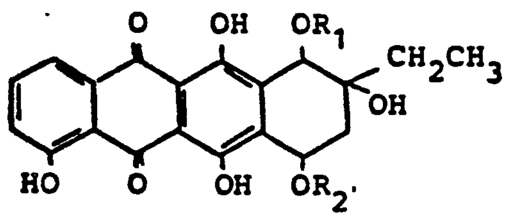

I,

wobei $R_1$ und $R_2$ verschieden sind und einer der Reste den Zuckerrest Roa der Formel

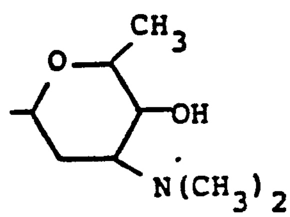

darstellt, während der andere Rest die Zuckerkombination

-Roa-Rod-Rod der Formel

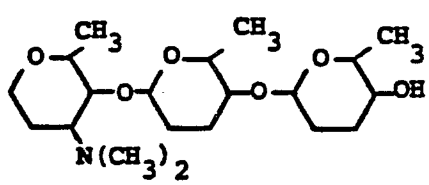

,

-Roa-Rod-Acu der Formel

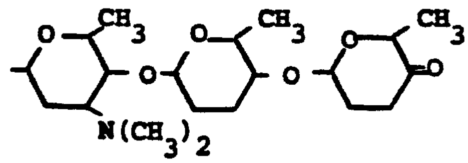

oder

-Roa-dF-CinA der Formel

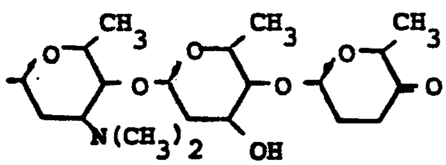

darstellt.

2. Verbindung der Formel I gemäß Anspruch 1, worin $R_1$ = Roa und $R_2$ = Roa-Rod-Rod bedeutet.

3. Verbindung der Formel I gemäß Anspruch 1, worin $R_1$ = Roa-Rod-Rod und $R_2$ = Roa bedeutet.

4. Verbindung der Formel I gemäß Anspruch 1, worin $R_1$ = Roa und $R_2$ = Roa-Rod-Acu bedeutet.

5. Verbindung der Formel I gemäß Anspruch 1, worin $R_1$ = Roa-Rod-Acu und $R_2$ = Roa bedeutet.

6. Verbindung der Formel I gemäß Anspruch 1, worin $R_1$ = Roa und $R_2$ = Roa-dF-CinA bedeutet.

7. Verbindung der Formel I gemäß Anspruch 1, worin $R_1$ = Roa-dF-CinA und $R_2$ = Roa bedeutet.

8. Verfahren zur Herstellung von Verbindung der Formel I gemäß Anspruch 1, dadurch gekennzeichnet, daß man in einer Verbindung der Formel II

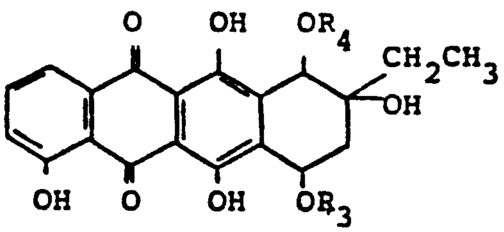

II,

worin einer der beiden Substituenten $R_3$ und $R_4$ eine der Zuckerkombinationen Roa-Rod-Rod, Roa-Rod-Acu oder Roa-dF-CinA und der andere Substituent eine der Zukkerkombinationen Roa-Rod-Rod, Roa-Rod-Acu, Roa-dF-CinA, Rod-dF=CinB oder Roa-dF-Rod darstellt, wobei

Roa Rhodosamin,

dF Desoxyfucose,

Rod Rhodinose,

Acu Aculose,

Cin A Cinerulose A,

Cin B Cinerulose B

darstellt und dF=CinB bedeutet, daß die beiden Zuckerbausteine durch eine zusätzliche Ätherbrücke neben der üblichen glykosidischen Bindung verknüpft sind, durch Behandlung mit einer Säure das endständige Disaccharid in einer der beiden Trisaccharidketten abspaltet.

9. Verfahren nach Anspruch 8, dadurch gekennzeichnet, daß man als Säure eine verdünnte starke Säure, vorzugsweise, 0,1 N-Salzsäure, Schwefelsäure oder Phos phorsäure oder eine verdünnte mittelstarke Säure, vorzugsweise 0,2 N-Ameisensäure oder 1 N-Essigsäure verwendet.

10. Verfahren nach Anspruch 8, dadurch gekennzeichnet, daß man die Säurebehandlung bei Raumtemperatur oder leicht erhöhter Temperatur, z.B. 37°C, durchführt.

11. Verwendung der Verbindungen gemäß Ansprüchen 1 bis 7 sowie ihrer physiologisch verträglichen Salze zur Herstellung von Arzneimitteln mit cytostatischer Wirkung.

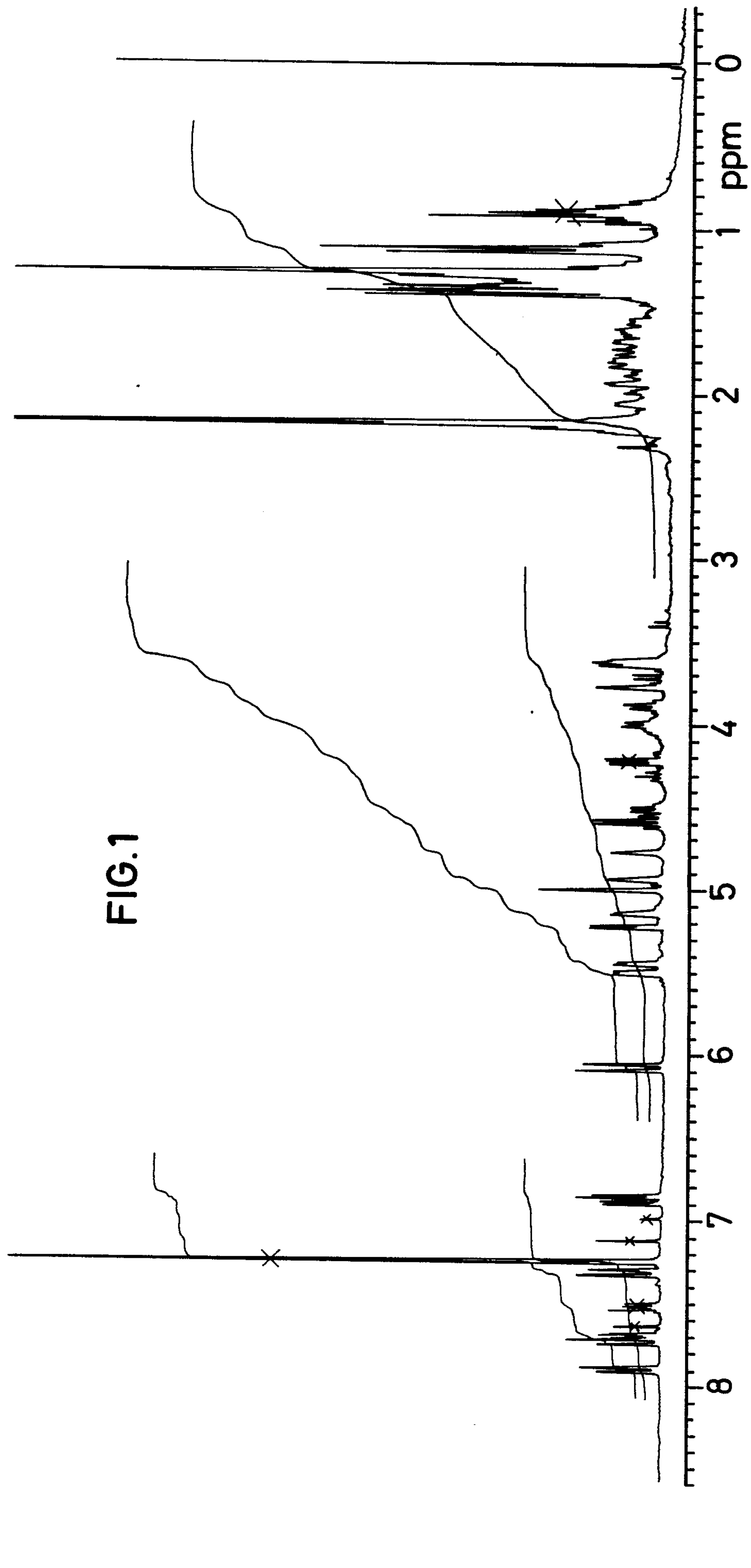


FIG.1

FIG.2

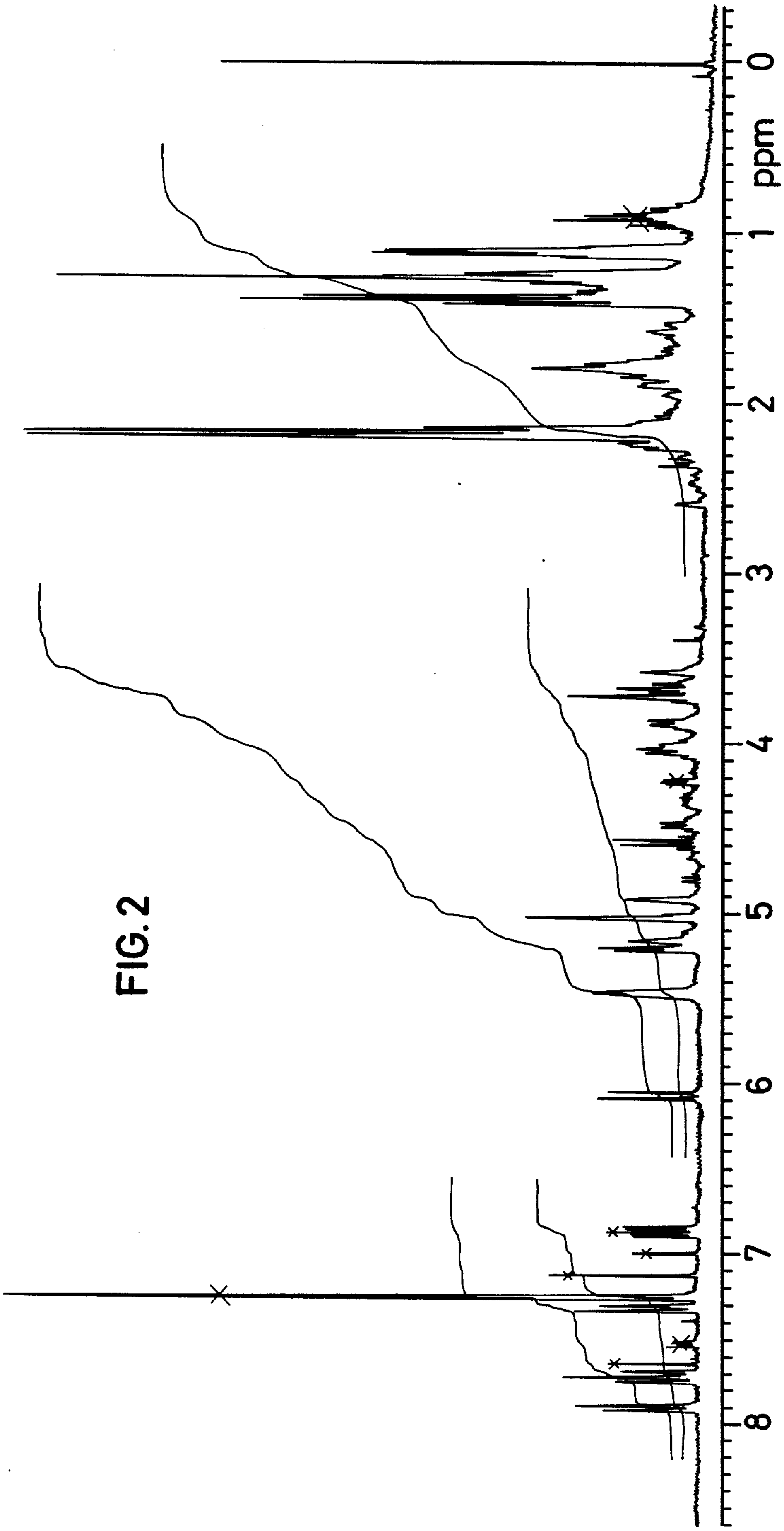

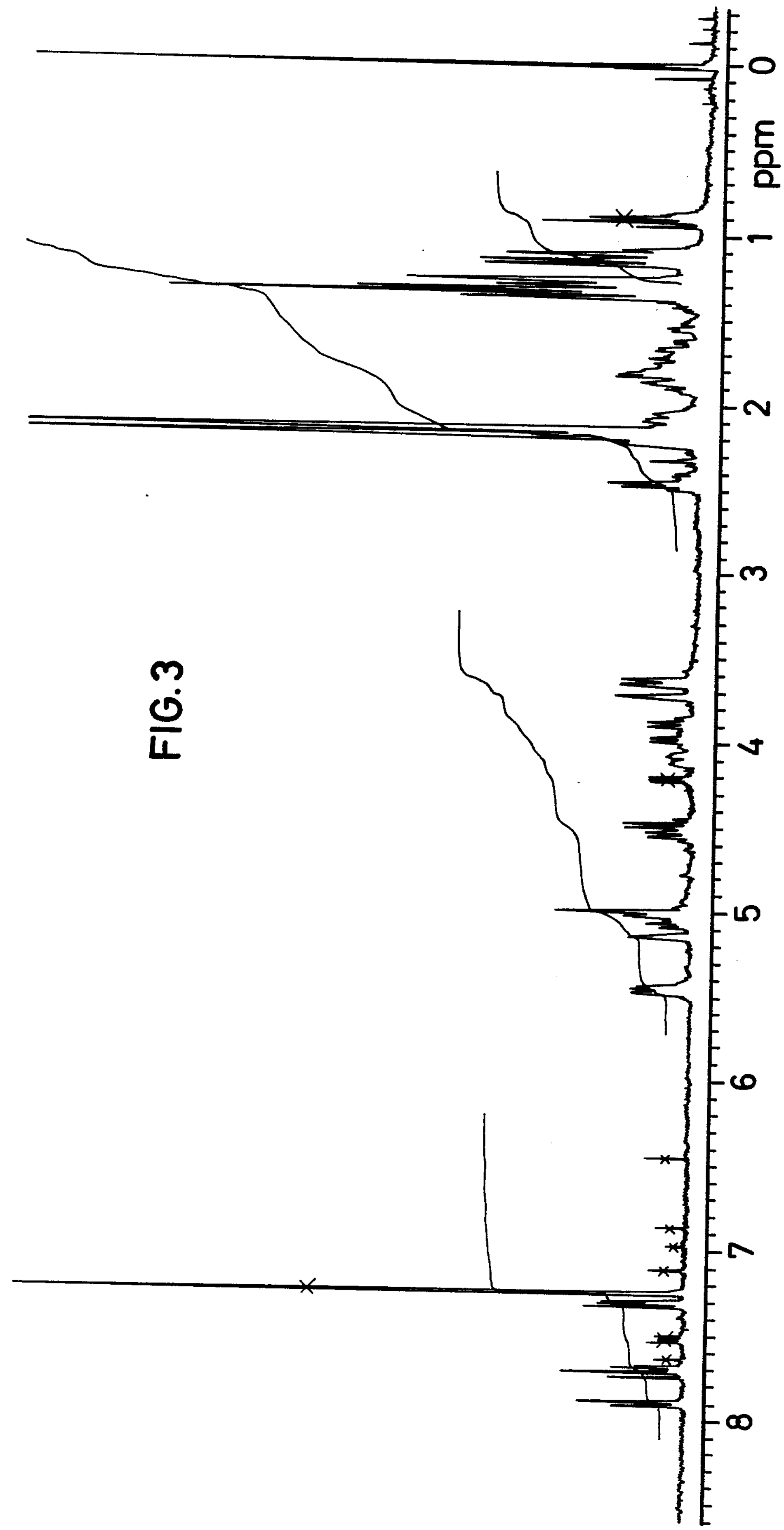


FIG.3